# EUROPEAN PATENT SPECIFICATION

(11) **EP 3 475 288 B1**
(45) Date of publication and mention of the grant of the patent: **03.11.2021**
(21) Application number: 17739322.0
(22) Date of filing: 22.06.2017
(51) Int. Cl.: C07D 495/04

(54) **PROCESS FOR THE PREPARATION OF HIGH-PURITY PRASUGREL**
VERFAHREN ZUR HERSTELLUNG VON HOCHREINEM PRASUGREL
PROCÉDÉ DE PRÉPARATION DE PRASUGREL DE HAUTE PURETE

(30) Priority: 23.06.2016 HU 1600389
(43) Date of publication of application: 01.05.2019
(73) Proprietor: Richter Gedeon Nyrt., 1103 Budapest (HU)
(72) Inventor: NEU, József, 1133 Budapest (HU); SZABÓ, Tamás, 2510 Dorog (HU); GARADNAY, Sándor, 1037 Budapest (HU)
(74) Representative: Kutzenberger Wolff & Partner
(86) International application number: PCT/IB2017/053721
(87) International publication number: WO 2017/221187

(56) References cited:
- WO-A1-2012/001486
- WO-A1-2012/052788
- WO-A1-2013/014295
- WO-A2-2009/062044
- WO-A2-2012/018791
- SAMPATH AALLA ET AL: "Process Improvements of Prasugrel Hydrochloride: An Adenosine Diphosphate Receptor Antagonist", ORGANIC PROCESS RESEARCH AND DEVELOPMENT, vol. 16, no. 2, 17 February 2012 (2012-02-17), pages 240-243, XP055398314, US ISSN: 1083-6160, DOI: 10.1021/op200325u

## Description

### FIELD OF THE INVENTION

This invention relates to a process, suitable for industrial scale manufacture, for the preparation of high-purity 5-[2-cyclopropyl-1-(2-fluorophenyl)-2-oxoethyl]-4,5,6,7-tetrahydrothieno[3,2-c]pyridine-2-yl acetate, prasugrel, of Formula (I).

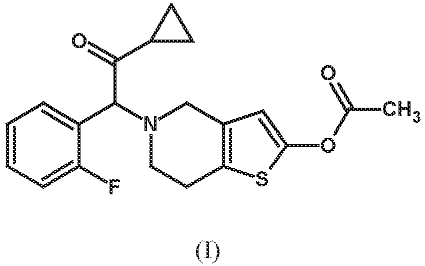

### STATE OF THE ART

Prasugrel is a pharmaceutical drug that acts as a platelet inhibitor and is used to prevent thrombosis, and was approved for the reduction of thrombotic cardiovascular events in people with acute coronary syndrome. Prasugrel was first described in JP2683479 B2 Japanese patent, European and USA equivalents: EP0542411 B1, US5288726 A. Prasugrel was first developed by Daiichi Sankyo Co. and produced by Ube Industries Ltd.

Several industrially applicable procedures are available for preparation of prasugrel. The synthesis generally is started from simple and relatively cheap molecules and in the multistep building of the end-product a number of impurities can occur. A main effort of the manufacturers is aimed at keeping back the quantities of these impurities under the necessary limits. The originator revealed a process for producing high-purity prasugrel in EP2003136 A4 and EP2367831 A1 with reducing quantity of an impurity, named OXTP. US2008/0176893 (Eli Lilly & Co.) describes methods for reducing the quantities of iminium salt and also OXTP 1 and OXTP 2 occurring during the storage of prasugrel tablets. The originator in EP2123656 B1 describe a method to keep back the quantity of an important impurity, 2-acetoxy-5-[5-chloro-l(2-fluorophenyl)-2-oxopentil]-4,5,6,7-tetrahydrothieno-[3,2-c] pyridine, chloropentyl impurity (also named CATP). In the present description the bromopentyl analogue occurs (here Formula (X)). WO2012/153348 (Glenmark Pharm. Ltd.) reveals a method for suppressing 1,5-dibromopentyl derivative (Formula (IX)), precursor of Formula (X). M. Cybulski et al published HPLC methods for different impurities: http://science24.com/paper/31051), http://science24.com/event/mknol2014/.

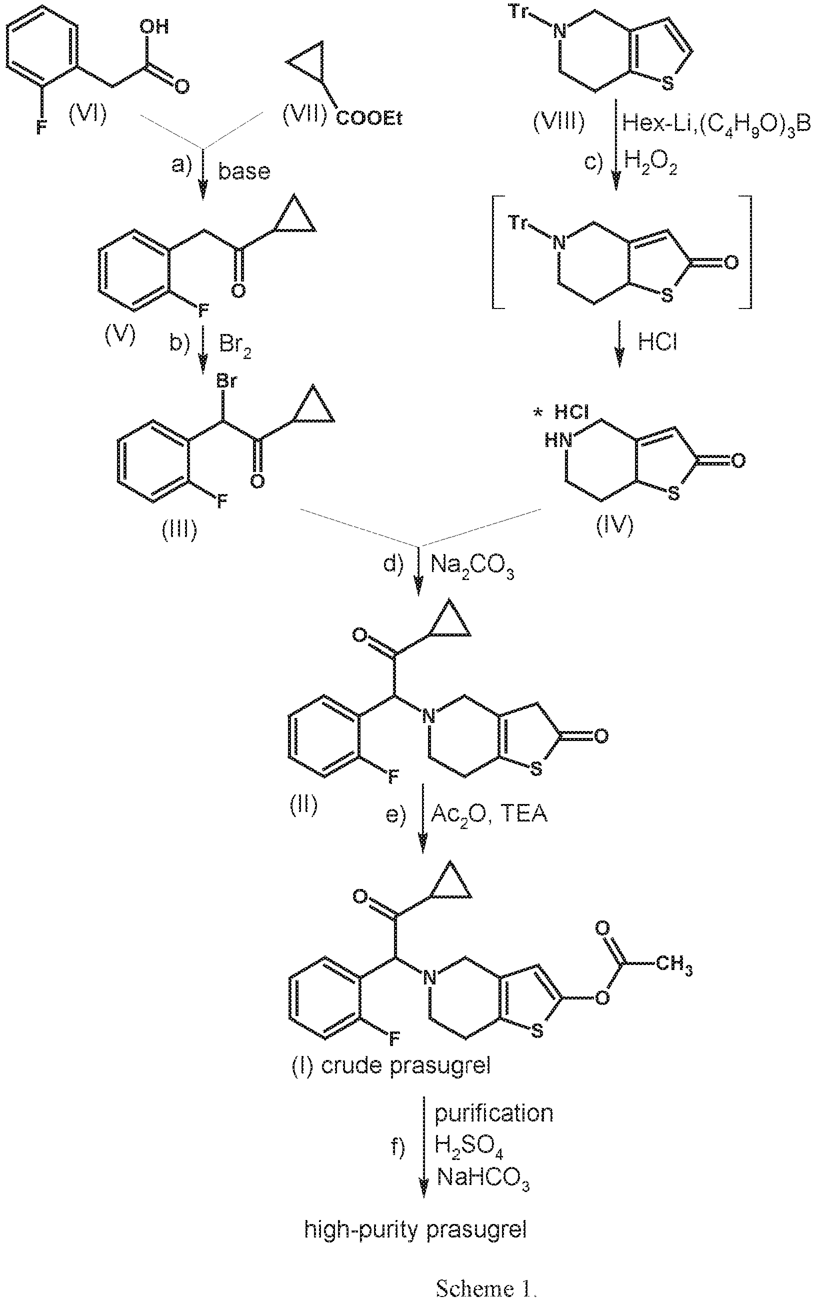

The health authorities prescribe particularly strict regulations for the potentially genotoxic impurities (European Medicines Agency, Guideline on the limits of genotoxic impurities). The limit concentration of a genotoxic impurity usually falls within the ppm range. The actual limit concentration is determined by the number of such impurities and the daily dose of the active ingredient.

A number of reaction schemes of known procedures are available from the literature. Scheme 1 is based on the procedures applied in EP0542411 B1, EP1298132 B1, US2007/0243243 A1, and similar schemes were used in several published documents.

### Step-a

Intermediate 1-cyclopropyl-2-(2-fluorophenyl)ethanone of Formula (V) can be prepared via Grignard compounds formed either from the aromatic component or from the other part of target molecule including the cyclopropyl fragment. US5288726 A1 describes method for forming a Grignard compound from the aromatic component and similar solutions are published in WO2009/122440 A1 (Torrent Pharm. Ltd.), WO2009/66326 A2 (MSN Labs. Ltd.), US2007/243243 (Cogentus Pharm. Inc.), WO2009/68923 A2 and WO2014/114964 A2 (EGIS Pharm. Ltd.) documents.

In WO20114/2918 A2; US2012/202066 A1 patent applications (MSN Labs Ltd) methods are revealed in which the Grignard compounds are formed from the cyclopropyl fragment.

Another type of methods are published in the WO2011/42918 A2 (MSN Labs Ltd) and WO2012/1486 A1 (Mayuka Labs Pvt. Ltd.) documents: not a coupling molecule part form a Grignard compound, but the isopropyl magnesium bromide is used as a base for preparation of the 1-cyclopropyl-2-(2-fluorophenyl)ethanone of Formula (V):

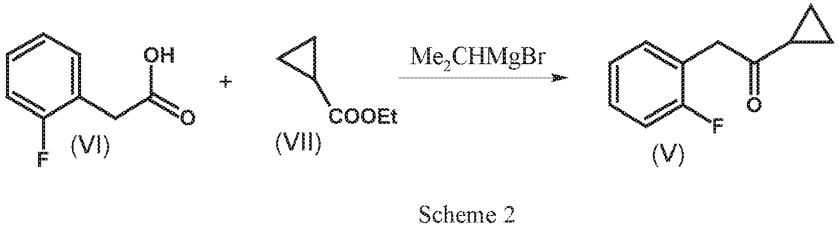

All in these procedures ether solvents are used that are liable for dangerous peroxidization and are the main source of the later impurities and moreover the ethers risk the freeze of the industrial scale reaction (start of the formation of Grignard compound is not instantaneous).

### Step-b

In patent application WO2009/122440 A1 (Torrent Pharm. Ltd) for the formation of 2-bromo-1-cyclopropyl-2-(2-fluorophenyl)ethanone of Formula (III) 1,3-dibromo-5,5-dimethyl-hidantoin is used as a halogenating agent and 2,2'-azo-bis-izobutyronitrile as catalyser. In the WO2012/52788 A1 patent application (EGIS Pharm. Ltd.) N-bromo-succinimide is used for bromination with p-toluene-sulphonic acidic catalysis. Similar method is used in patent application WO2012/1486 A1 (Mayuka Labs. PVT. Ltd.): dibenzoyl-peroxide is applied as a catalyser. According to the method of WO2013/14295 A (Labs. Lesvi) the 2-bromo-1-cyclopropyl-2-(2-fluorophenyl)ethanone of Formula (III) is obtained with halogenation by elemental bromine.

WO2012/153348 PCT application (Glenmark Pharm. Ltd.) revealed a method for reducing quantity of impurities. Using a quaternary ammonium-bromide in tetrahydrofuran (THF) and methanol for bromination yields a product with 1-2%, and 0.5% dibromo derivatives. However, these quantities are still far above the acceptable limits.

### Step-c

The 5,6,7,7a-tetrahydrothieno[3,2-*c*]pyridine-2(4*H*)-one hydrochloride product of Formula (IV) can be prepared according to Scheme 3 reacting the N-tritil-4,5,6,7-tetrahydrothieno[2,3-c]pyridine of Formula (VIII) with hexyl-lithium, tributyl-borate and hydrogen-peroxid in THF via a (generally isolated) intermediate (called tritiloxothieno-pyridine) from which Formula (IV) is formed with hydrogen chloride.

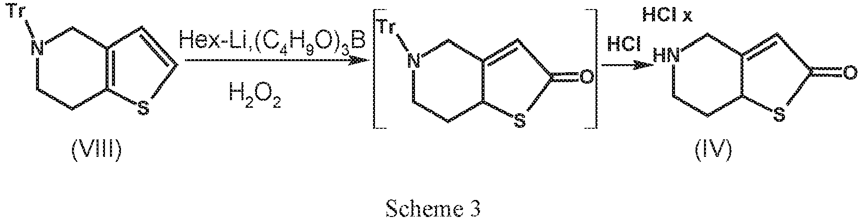

Table 1 summarises data of known procedures. (Each referenced procedures published only laboratory scale data.)

**Table 1:**

| | Solvent | Addition HexLi °C | Warming °C | Addition Borate °C | Warming °C | Addition Peroxid °C | Yield % | HexLi excess % |
|---|---|---|---|---|---|---|---|---|
| US4740510A (Sanofi S.A.) | THF | 0 | | -20 | | -40 | 64 | 120 |
| WO201177173 (EGIS Pharm.) | THF | -40 | 10 | -40 | 10 | -40 | 92 | 150 |
| WO20121486 (Mayuka Labs.) | 2 THF: 5Toluene | -5 | 10 | -5 | 10 | 20 | 76 | 148 |
| WO201142918 (MSN Labs.) | THF | 0-5 | 10 | 0-5 | 10 | 0-5 | 85 | 157 |
| Reproducton Richter | THF | 0-5 | 10-15 | 0-5 | 10-15 | 0-5 | 85-90 | 157 |

### Step-d

According to US5288726 A patent application (Examples 12, 34) 5-[α-cyclopropylcarbonyl-2-fluorobenzyl)-2-oxo-2,4,5,6,7,7a-hexahydrothieno[3,2-*c*]pyridine of Formula (II) can be obtained as a result of a condensation reaction from 2-bromo-1-cyclopropyl-2-(2-fluorophenyl)ethanone of Formula (III) and 5,6,7,7a-tetrahydrothieno[3,2-*c*]pyridine-2(4*H*)-one hydrochloride of Formula (IV). In Example 34 the hydrochloride salt of Formula (II) is prepared by crystallization from diethyl ether. This reaction was applied in several later publications, as well.

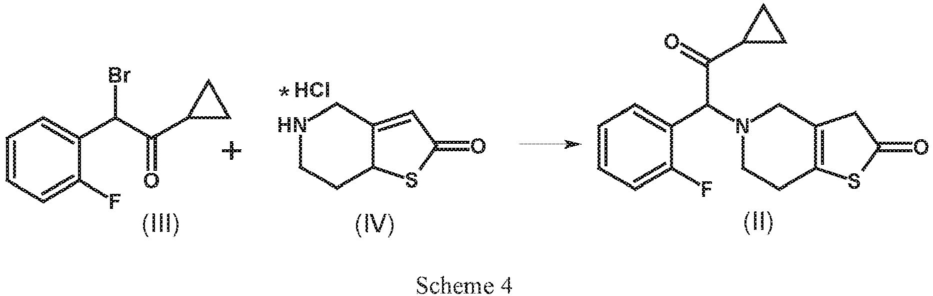

### Step-e

According to US5288726 A, Example 23, the crude 5-[2-cyclopropyl-1-(2-fluorophenyl)-2-oxoethyl]-4,5,6,7-tetrahydrothieno[3,2-*c*]pyridine-2-yl acetate (prasugrel) of Formula (I) can be prepared from the 5-[α-cyclopropylcarbonyl-2-fluorobenzil)-2-oxo-2,4,5,6,7,7a-hexahydro-thieno[3,2-*c*]pyridine oxo-compound of Formula (II). The crystals were obtained from diisopropyl ether.

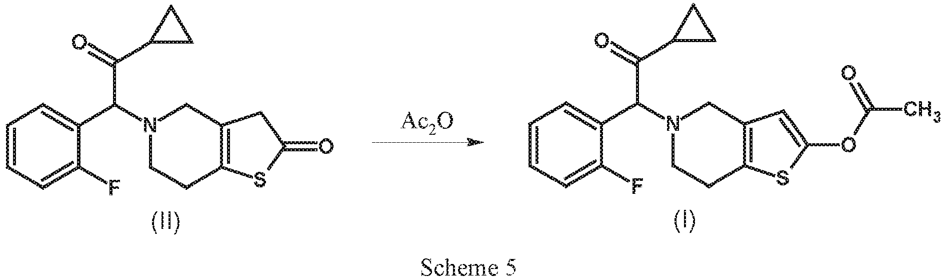

Similar but improved methods are used in a number of later patent documents, as well.

### Step-f (purification of prasugrel)

An obvious purification method of crude prasugrel includes salt-forming, crystallization of the salt, filtering the crystals, solving and reconverting it to base, and crystallising, filtering again. Even this double crystallisation procedure is effective only if all the preceding steps were carried out with well established circumstances.

In EP2003136 A1 (Daiichi Sankyo Co.) a method was reported to eliminate a significant contamination problem, changing the preceding procedure, an intermediate (OXTP) was removed from prasugrel by crystallisation, thus the concentration of the unwanted component was reduced to 2-300 ppm.

EP2112155 B1 (Sandoz AG) and EP2325187 A1 (Lunan Pharm. Co Ltd.) revealed examples for prasugrel sulphate salt forming.

WO2012/052788 identifies 5-[5-bromo-1-(2-fluorophenyl)-2-oxopentyl]-4,5,6,7-tetrahydrothieno[3,2-*c*]pyridine-2-yl acetate (here Formula (X)) as an impurity.

### SUMMARY OF THE INVENTION

The present invention provides an industrially applicable economic process for the preparation of high-purity 5-[2-cyclopropyl-1-(2-fluorophenyl)-2-oxoethyl]-4,5,6,7-tetrahydrothieno[3,2-c]pyridine-2-yl acetate, prasugrel, of Formula (I). In all steps of our process (Scheme 1) from the beginning such conditions are used that serve for the restriction of all the known impurities in the end-product.

According to the present procedure starting from the 1-cyclopropyl-2-(2-fluorophenyl) ethanone of Formula (V) each reaction product is prepared strictly excluding the ether solvents (diethyl ether, THF, diisopropyl ether, etc.).

With adjusting the conditions of bromination of 1-cyclopropyl-2-(2-fluorophenyl)ethanone of Formula (V) the quantity of a by-product leading to the impurity of Formula (X) is restricted.

In contrast with generally applied literature procedures, in our method the 5,6,7,7a-tetrahydrothieno[3,2-*c*]pyridine-2(4*H*)-one hydrochloride of Formula (IV) is prepared without ether solvents, thus avoiding the formation of by-products in high concentration, especially during the process scale-up.

The present invention provides an improved cleaning operation. In a cleaning reaction the impurity of Formula (X) is converted to an easily removable high-polarity compound.

### DESCRIPTION OF THE INVENTION

In our process the crude prasugrel is prepared on the way schematically outlined in Scheme-1 basically according to the method published in US5288726 A (Example 23, compound 190). Surprisingly experienced that especially in large-scale production, one of the main causes of piling up the impurities is the use of ether solvents, consequently, in each step in this procedure ethers are strictly excluded. Avoiding ethers resulted new conditions for production of intermediates: in step-a LiH is used as a base in ether-free media, the execution of coupling reaction in step-d to form 5-[α-cyclopropylcarbonyl-2-fluorobenzyl)-2-oxo-2,4,5,6,7,7a-hexahydrothieno[3,2-*c*]pyridine of Formula (II) is new and unique, and the purification of the active is developed in a new way, as well. Conditions were determined so that each step from the beginning contributes to minimizing the impurity content of the end-product.

### Step-a

We started from the methods of WO20l1/42918 A2, WO2012/1486 A1 patent applications, where for preparing the 1-cyclopropyl-2-(2-fluorophenyl)ethanone of Formula (V) not a coupling molecule part forms a Grignard compound, the i-propyl-magnesium-bromide is used only as a base. We checked whether this reaction can be carried out in non-ether solvents with other basic compounds. Surprisingly, it was observed, that the use of Grignard compounds, that generally entails the use of ethers, too, is not necessary. The reaction takes place in other solvents, e.g. in a mixture of toluene and dimethyl sulphoxide in the presence of NaH, moreover, with optimising the ratio of components the yield is excellent.

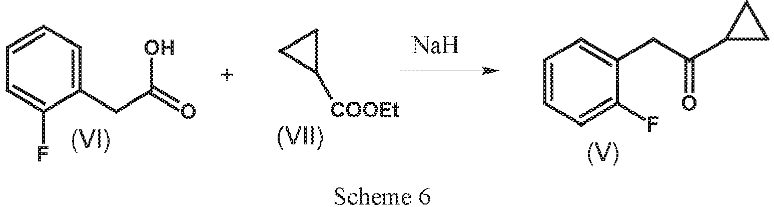

The basicity of sodium-hydride can be well regulated with the composition of the solvent mixture. In pure dimethyl sulphoxide the 1-cyclopropyl-2-(2-fluorophenyl)ethanone of Formula (V) practically becomes a minor component. If only toluene is used the reaction even does not take place. The yield grows when dimethyl sulphoxide content of the solvent mixture exceeds 50 %, and the purity of the product improves with the decrease of dimethyl sulphoxide content. The mixture of toluene- dimethyl sulphoxide can be used from ratio 1:1 to 98:2, the 9:1 is preferable, and the 95:5 ratio the most preferable according to our data. The reaction step implemented this way is more economic than the known other methods, ensures more safe industrial scale preparation, moreover it is robust: with a bulk scale of 20 kg a high pure intermediate is obtained with a yield of 87%.

### Step-b

We reproduced and studied the procedures published in WO2009/122440 A1, WO2012/52788 A1, WO2012/1486 A1 and WO2013/14295 A, and it could be stated that the quantity of by-product 1,5-dibromo-1-(2-fluorophenyl)pentane-2-oneof Formula (**IX**) grows significantly with the industrial scale-up. While at 5-10 g scale its concentration is only 0.3%, at 250-300 g level 0.59%, and at 4-5000 g scale 1.6%. Impurity of Formula (**IX**) requires special attention, the precursor of 5-[5-bromo-1-(2-fluorophenyl)-2-oxopentyl] -4,5,6,7-tetrahydrothieno[3,2-c]pyridine-2-yl acetate, the bromo-pentyl impurity of Formula **(X)** which cannot be removed from the end-product with crystallisation because of solubility features.

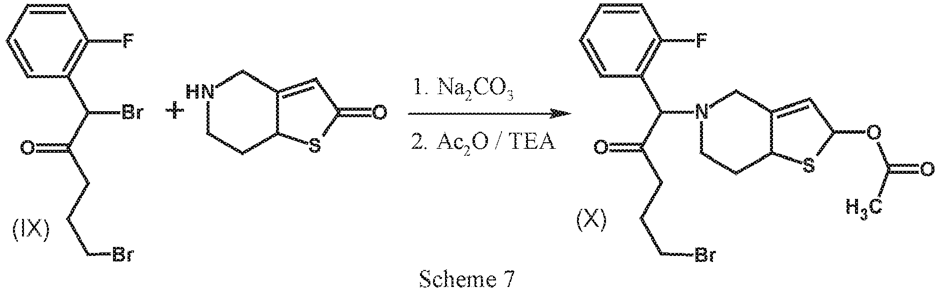

According to our results the products of bromination with N-bromo-succinimide or 1,3-dibromo-5,5-dimethyl-hidantoin contains 1-5% undesired 1,5-dibromo-1-(2-fluorophenyl) pentane-2-one of Formula (IX), even in laboratory circumstances.

Our analytical investigations with structure elucidation and coupled separation techniques proved the possible appearance of other trace impurities, as well:
Since even with reproducing the bromination method of WO2012/153348 A2 still 5-10,000 ppm potential impurities occur, we studied the possibilities for preventing the formation of 1,5-dibromo-1-(2-fluorophenyl)pentane-2-one of Formula (IX). It was surprisingly experienced that with optimising the reaction temperature, molar ratio, and the other conditions of the process, it is possible to produce 2-bromo-1-cyclopropyl-2-(2-fluorophenyl)ethanone of Formula (III) containing not more than 0,05% 1,5-dibromo-1-(2-fluorophenyl)pentane-2-one of Formula (IX), even at a half-industrial scale (17 kg). For the best results the bromine is applied in equivalent quantity, the reaction temperature is 20-25°C, the working-up temperature is kept between 0-5°C, and the formed hydrogen bromide is absorbed with a base.

### Step-c

We reproduced Step-c both in laboratory and half-industrial (10 kg) scale with conditions summarised in Table 1. In spite of the same time and temperature of the addition, the concentration of the unknown by-product of Formula (XI), grew from the 2-3% of laboratory scale to 18-20% in case of half-industry scale, and even two more by-products Formulae (XII, XIII) appeared, according to coupled separation techniques and structure elucidation:

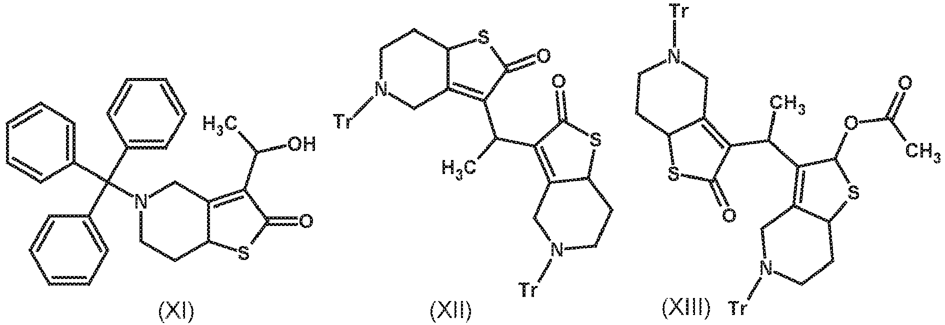

Although it is known from literature (M. Schlosser, Organometallics in Syntehesis, John Wiley & Sons 1994, W. Weiß, EP0753520 B1) that at higher temperature the hexyl-lithium reacts with THF, at the temperature of 0-5°C such a reaction was not expected, even in such a high quantity.

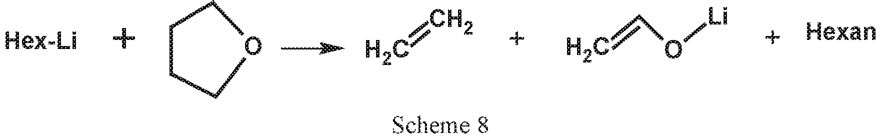

The by-products not only decrease the yield, but prevent achieving the Pharmacopoeial and higher qualities. Some producers use extreme low temperature which can reduce the quality problems but increases the expenditures. In our studies we tried to prevent the unwanted reactions, to avoid extreme low temperature and the complicated temperature cycles (Table 1) used, so far, according to the literature. Especially in larger scale, a further advantage is, if the work up of reaction mixture does not need the application of an extra solvent (this unavoidable in case of e.g. the water-miscible THF).

We studied the possibilities of avoiding ether solvents. Taking into account the solubility properties of hexyl-lithium and the N-trityl -4,5,6,7-tetrahydrothieno[2,3-c]pyridine of Formula (VII), among others, toluene was tried, and we learnt that only in toluene the reaction does not take place at all. It is known from literature that the ether solvents disrupt the hexyl-lithium aggregates in hexane, thus make it more reactive. For increasing the reactivity N,N,N',N'-tetramethyl-ethylene-diamine (in short: TMED) can also be used in THF (J.Am.Chem.Soc., 92,4664, 1970). It is also known from the literature that the this way activated butyl-lithium (can be regarded closely similar to hexyl-lithium) is able to metallate benzene, toluene, etc. with forming the appropriate aryl lithium salt (J.Am.Chem.Soc.,88,460, 1966). Surprisingly, it was found that this reaction step can also be carried out without THF or other ethers. If N-trityl -4,5,6,7-tetrahydrothieno[2,3-c]pyridine of Formula (VII) is solved in toluene followed by addition of TMED, and dropwise addition of a solution of hexyl-lithium in hexane at temperature 0-5 °C, lithium exchange reaction can take place. The formed N-trityl -4,5,6,7-tetrahydrothieno[2,3-c]pyridine-lithium salt can be reacted with tributyl-borate, after its oxidation the formed, not isolated, intermediate is decomposed by hydrogen chloride, as a result 5,6,a-tetrahydrothieno[3,2-c]pyridine-2(4*H*)-one hydrochloride of Formula (IV) is obtained with a good yield.

As a further advantage, this procedure does not require an extra solvent for the extraction. The procedure makes possible the economic litiation at the generally used temperature range of 0-5 °C, avoiding the cooling-warming cycles generally used according to the literature (see Table 1). Avoiding isolation of the intermediate N-trityl -4,5,6,7-tetrahydrothieno[2,3-c]pyridine-2-on decreases the expenditures, and as a result of suppression of the undesired by- reactions, after removing of the protecting group, 5,6,7,7a-tetrahydrothieno[3,2-c]pyridine-2(4H)-one hydrochloride of Formula (IV) is obtained with an appropriate purity.

### Step-d and Step-e

We used these two steps merged according to known methods from literature but with excluding the use of ether solvents.

### Step-f

The previous procedure steps generally ensures such a pure crude prasugrel that in this purification step a salt forming with crystallisation, filtering off the salt crystals, the repeated forming of prasugrel base and filtering off the base ensures the highly pure prasugrel end-product. A further purification method is inserted into the purification step.

This method is based on the surprising recognition that the impurity 5-[5-bromo-1-(2-fluorophenyl)-2-oxopentyl ]-4,5,6,7-tetrahydrothieno[3,2-*c*]pyridine-2-yl acetate of Formula (X) can be taken into such a reaction, even in the presence of the rather sensitive prasugrel (active ester!), in which the easily removable high polarity quaternary iodide compound of Formula (Xb) is formed.

It was recognised that according to Scheme 9 the two-step conversion is preferable, first bromine is cautiously exchanged to iodine, since this iodine-carbon bond has better reaction ability to form water-soluble quaternary salt. For this purpose the acetonitrile, the ketones with small number of carbon atoms (≤6), e.g. acetone, methyl-ethyl-ketone, methyl-isobutyl-ketone, etc. are suitable solvents.

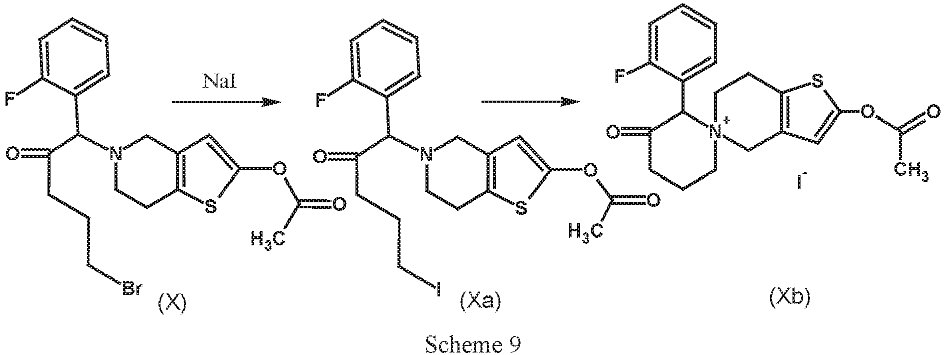

The reaction can take place at a low temperature, but warming speeds it up. At room temperature 24 h, at 40°C 6 h is necessary to accomplish the conversion according to Scheme 9.

Preferably the reaction is carried out in a water-miscible solvent, since with diluting the reaction mixture with water the purified product is precipitated, and the impurity, converted to quaternary ammonium iodide compound, remains in the aqueous phase. Yield of this special cleaning operation: 94-96% bromopentyl impurity decontaminated crude prasugrel.

The bromopentyl-impurity-free crude prasugrel is purified by forming and crystallising and separating sulphate salt, reconverting the salt into prasugrel base and crystallising and separating the base again. In our conditions a hygroscopic mixed sulphuric salt is obtained, according to titrimetric results it is a mixture of prasugrel-hydrogen-sulphate and prasugrel-sulphate. (Homogeneous crystal form is not necessary in this phase.) Without complete drying, the separated sulphate salt is solved in dichloromethane handled with sodium-hydrogen-carbonate solution then the solvent is changed and with a further crystallization and separation the high-purity prasugrel base, free from genetic and other impurities, is obtained.

### EXAMPLES

The following examples are only illustrative ones and are provided to enable one skilled in the art to practice the invention. The examples should not be read as limiting the scope of the invention.

### Example 1: Preparation of 1-cyclopropyl-2-(2-fluoroophenyl)ethanone of Formula (V)

A solvent mixture is prepared from 228 l toluene and 12 1 dimethyl sulphoxide. To 120 l of so-prepared solvent mixture 21.12 kg sodium-hydride is measured, then with intensive stirring, a solution of 24.66 kg 2-fluoro-phenil acetic acid in 80 1 solvent mixture is added. After completion of the addition, the temperature of the reaction mixture is elevated to 95-100°C, then 30 l solvent mixture and 23.2 l (0.195 mol) cyclopropan-carboxylic acid-ethyl ester are added. Having elevated the temperature to 108-110°C, the reaction mixture is stirred for 1.5 hours at this temperature. To the reaction mixture 24 l dimethyl sulphoxide is added then the mixture is cooled and added into 240 l water. The emulsion is stirred for 3 hours at a temperature of 20-25°C. The phases are separated and the aqueous phase is washed with 40 1 toluene. The united organic phase is washed with distilled water then it is evaporated from an oil bath at a reduced pressure of 0.9 Pa and at 130-138°C, and dried.

25.1 kg title compound is obtained (yield: 88 %).

### Example 2: Preparation of 2-bromo-1-cyclopropyl-2-(2-fluorophenyl)ethanone of Formula (III)

To 230 l methanol 17.82 kg 1-cyclopropyl-2-(2-fluorophenyl)ethanone is measured. To the reaction mixture with continuous stirring in a period of 2-2.5 hour, 15.98 kg bromine is added gradually, the reaction mixture is cooled to 0-5°C temperature, then 230 1 dichloromethane and 8,4 kg sodium-hydrogen-carbonate, and 230 1 water are added. The phases are separated and the aqueous phase is washed with 100 l dichloromethane. The combined organic phase is washed with 100 1 water, dried and evaporated at reduced pressure.

26.11 kg title compound is obtained (corrected with the content: 24.62 kg, yield: 95.8%)), the concentration of 1,5-dibromo-1-(2-fluoropheny)pentane-2-one of Formula (**IX**) is not more than 0.05% (with capillary gas-chromatography).

### Example 3: Preparation of 5,6,7,7a-tetrahydrothieno[3,2-c]pyridine-2(4H)-one hydrochloride of Formula (IV)

To 165 1 toluene 26.14 kg N-tritil-4,5,6,7-tetrahydrothieno[2,3-c]pyridine and 15.5 1 N,N,N',N'-tetramethyl-ethylene-diamine is measured. With stirring, the obtained solution is cooled at 0±3°C then 50.0 1 hexyl-lithium of 2.47 mol/l concentration is added gradually in a 30-60 min period. After completion of the addition the yellow solution is stirred at 0±3°C for 30 min, then a mixture of 37.2 l tributyl-borate and 37.2 l toluene is added, then the solution is stirred for another 1 hour at 0±3°C. To the reaction mixture 30.7 1 hydrogen-peroxide of 30% concentration is added. The temperature is allowed to elevate to 20-25°C and the mixture is stirred at this temperature for a further 1 hour. The toluene phase is washed first with 50 l, then 3×30 l water. The organic phase is dried, evaporated then 110 l acetone is added. 7.0 1 36% hydrogen chloride is added to the suspension. After 1 hour stirring the product is filtered out and washed with 1×20.0 1 acetone and dried.

Thus 11.31 kg title compound is obtained (yield: 86, 1%).

### Example 4: Preparation of crude prasugrel base of Formula (I)

To 52 1 methyl-isobutyl-ketone 17.8 kg anhydrous sodium-carbonate and 10.0 kg 96% 5,6,7,7a-tetrahydrothieno[3,2-*c*]pyridine-2(4*H*)-one hydrochloride of Formula (IV) are measured then 13.4 kg 96% 2-bromo-1-cyclopropyl-2-(2-fluorophenyl)ethanone of Formula (III) is added. The mixture is stirred for 10 h at 40°C then cooled, and the inorganic compounds are filtered off. To the filtrate 300 g dimethylamino-pyridine and 22.4 l triethylamine are added, cooled to 0-5°C then 11.0 l acetic anhydride is added dropwise. After 2 h, to the mixture 40 1 ethyl acetate then 40 1 water are added dropwise. After separation the organic phase is dried then evaporated to dryness. 2×40 l ethanol is poured onto the reminder then evaporated. The oily remainder is solved in 40 l ethanol. The crystalline material is stirred first at 20-25°C for 1 h then at 0-5°C for further 1 h then filtered, washed with 8 l cool ethanol and dried.

Thus 11.4 kg crude prasugrel base is obtained. (Yield: 61 %.)

### Example 5: Preparation of crude prasugrel base free from bromopentyl impurity of Formula (X)

To 44.0 1 acetone 11.0 kg crude prasugrel base and 1.1 kg sodium-iodide are measured. The mixture is heated to 60°C, stirred for 6 hours at this temperature then 44.0 1 water is added. The mixture is cooled to 0-5°C then the purified crude prasugrel is filtered off and washed with water and dried.

Thus 10.5 kg title compound is obtained. (Sum of impurities: 0.48 %., each individual impurity is less than 0.19 % by GC.)

### Example 6: Preparation of high-purity prasugrel base

To 150.0 l acetone 10.0 kg prasugrel base free from bromopentyl impurity of Formula (X) is measured. The solution obtained is cooled to -5 - -10°C and a freshly made solution of 1.5 l 95-97% sulphuric acid and 40.0 1 acetone is added dropwise. After stirring for 3 h the product is filtered out and washed. The wet sulphate salt product is solved in 45 1 dichloromethane then the solution is washed with 60 1 saturated sodium-hydrogen-carbonate solution. After drying the organic phase is evaporated, the solvent is changed for ethanol. The residue is crystallised from 33 1 ethanol, filtered and dried.

8.6 kg high-purity prasugrel base is obtained (Sum of impurities: 0.13 %, each individual impurity is less than 0.1 %, concentration of compound of Formula (X) is less than 150 ppm. by HPLC.)

## Claims

1. A process for the preparation of 5-[2-cyclopropyl-1-(2-fluorophenyl)-2-oxoethyl]-4,5,6,7-tetrahydrothieno[3,2-c]pyridine-2-yl acetate (prasugrel) of Formula (I) 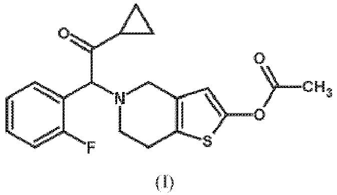 including the following steps:
**a)** (2-fluorophenyl)-acetic acid of Formula (VI) is reacted in the presence of a base with cyclopropane carboxylic acid ethyl ester of Formula (VII), 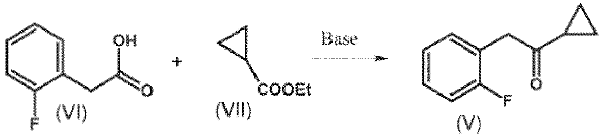
**b)** then the obtained 1-cyclopropyl-2-(2-fluorophenyl)ethanone of Formula (V) is brominated to obtain 2-bromo-1-cyclopropyl-2-(2-fluorophenyl)ethanone base of Formula (III), 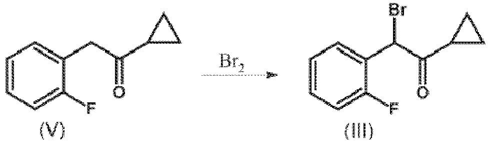
**c)** N-trityl-4,5,6,7-tetrahydrothieno[2,3-c]pyridine of Formula (VIII) is reacted with hexyllithium, then tributyl-borate, finally with hydrogen-peroxide, 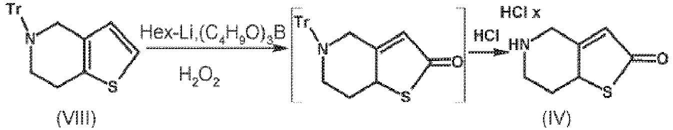 to form 5,6,7,7a-tetrahydrothieno[3,2-c]pyridine-2(4H)-one hydrochloride of Formula (IV),
**d)** the 2-bromo-1-cyclopropyl-2-(2-fluorophenyl)ethanone base of Formula (III) in the presence of sodium carbonate is reacted with 5,6,7,7a-tetrahydrothieno[3,2-*c*]pyridine-2(4*H*)-one hydrochloride of Formula (IV), 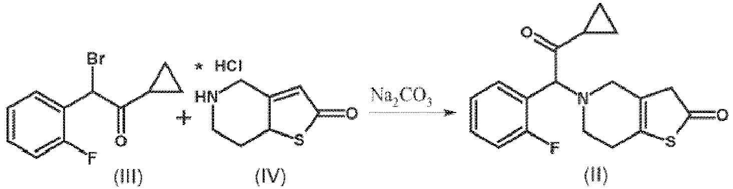 to obtain the 5-[α-cyclopropyl carbonyl-2-fluorobenzyl)-2-oxo-2,4,5,6,7,7a-hexahydro-thieno[3,2-c]pyridine of Formula (II),
**e)** the compound of Formula (II) is acetylated and the so obtained crude 5-[2-cyclo-propyl-1-(2-fluorophenyl)-2-oxoethyl]-4,5,6,7-tetrahydrothieno[3,2-c]pyridine-2-yl acetate (prasugrel) of Formula (I) is prepared, 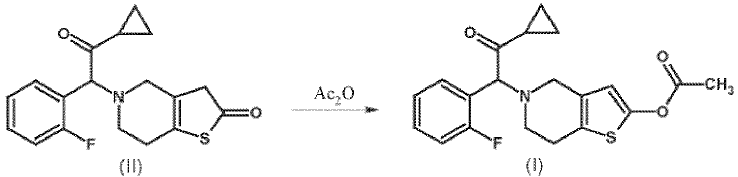
**f)** finally in a purification step the crude prasugrel is converted into a purified prasugrel product by removing a bromopentyl impurity of Formula (X) 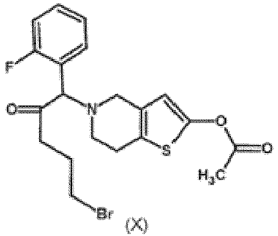 from 5-[2-cyclopropyl-1-(2-fluorophenyl)-2-oxoethyl]-4,5,6,7-tetrahydrothieno[3,2-c]-pyridine-2-yl acetate (prasugrel) of Formula (I) 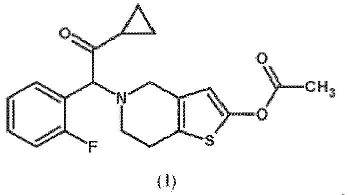 wherein said impurity is converted to an easily removable high-polarity quaternary ammonium iodide of formula (Xb) 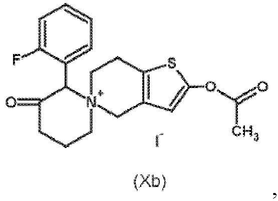 and
wherein the use of ether solvents are excluded from each step of the process.

2. The process according to claim 1, wherein bromopentyl impurity of Formula (X) is solved in organic solvent, then reacted with an excess of sodium iodide, with halogen atom change converted to a compound (Xa), then to a water-soluble compound of Formula (Xb), 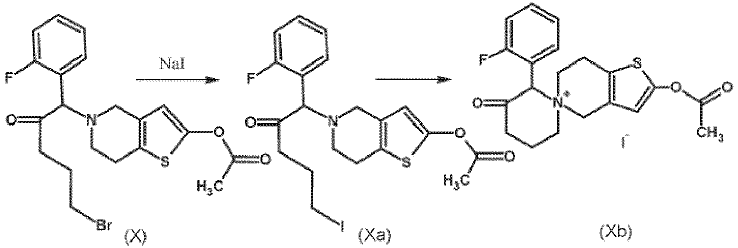 finally with water-addition the purified prasugrel is filtered out and dried.

3. The process of claim 2, wherein the reaction with sodium iodide is carried out in an aprotic solvent.

4. The process according to claim 3, wherein the aprotic solvent is acetone.

5. The process according to any of the preceding claims wherein the reaction of step **a)** is carried out in the presence of sodium hydride base.

6. The process according to any of the preceding claims wherein the reaction of step **a)** is carried out in a mixture of toluene and dimethyl sulphoxide solvents.

7. The process according to claim 6, wherein the mixture of toluene and dimethyl sulphoxide solvents is in a mixture of 95:5 ratio.

8. The process according to any of the preceding claims wherein the bromination of step **b)** is carried out with equivalent quantity of bromine at a temperature in a range of 24-27 °C, the formed hydrogen bromide is removed at a temperature of 0-5 °C with using a base in the aqueous phase, and 2-bromo-1-cyclopropyl-2-(2-fluorophenyl)ethanone is obtained with the evaporation of the organic phase.

9. The process according to any of the preceding claims wherein the lithiation according step **c)** is carried out in toluene using tertiary amine catalysation.

10. The process according to claim 9, wherein the lithiation according step **c)** is carried out in the presence of N,N,N',N'-tetramethyl ethylene-diamine.

11. The process according to claim 9 or 10, wherein the lithiation according step **c)** is carried out at a temperature in the range of 0-5 °C.

## Patentansprüche

1. Ein Verfahren zur Herstellung von 5-[2-Cyclopropyl-1-(2-fluorphenyl)-2-oxoethyl]-4,5,6,7-tetrahydrothieno[3,2-c]pyridin-2-ylacetat (Prasugrel) der Formel (I) 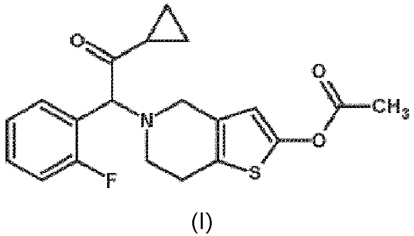 umfassend die folgenden Schritte:
**a)** (2-Fluorphenyl)-Essigsäure der Formel (VI) wird in Gegenwart einer Base mit Cyclopropancarbonsäureethylester der Formel (VII) umgesetzt, 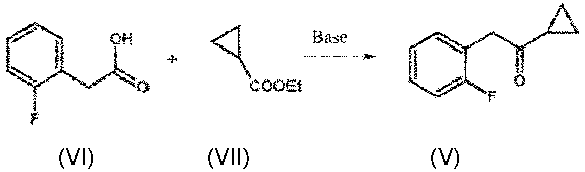
**b)** dann wird das erhaltene 1-Cyclopropyl-2-(2-fluorphenyl)ethanon der Formel (V) bromiert, um 2-Brom-1-cyclopropyl-2-(2-fluorphenyl)ethanon Base der Formel (III) zu erhalten, 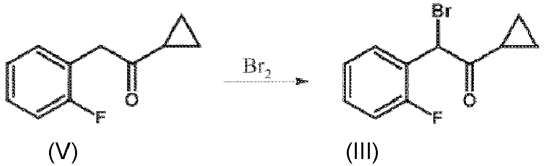
**c)** N-Trityl-4,5,6,7-tetrahydrothieno[2,3-c]pyridin der Formel (VIII) wird mit Hexyllithium, dann mit Tributylborat, schließlich mit Wasserstoffperoxid umgesetzt, 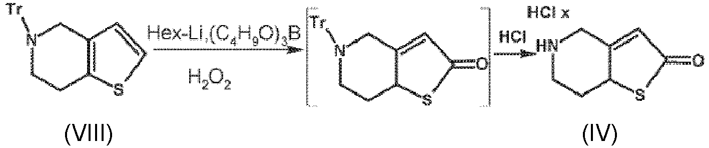 um 5,6,7,7a-Tetrahydrothieno[3,2-c]pyridin-2(4H)-on Hydrochlorid der Formel (IV) zu bilden,
**d)** die 2-Brom-1-cyclopropyl-2-(2-fluorphenyl)ethanon Base der Formel (III) wird in Gegenwart von Natriumcarbonat mit 5,6,7,7a-Tetrahydrothieno[3,2-*c*]pyridin-2(4*H*)-on Hydrochlorid der Formel (IV) umgesetzt, 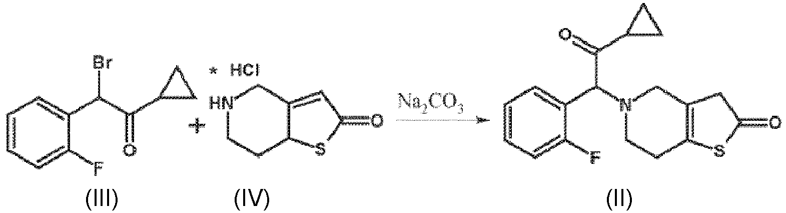 um das 5-[α-Cyclopropylcarbonyl-2-fluorbenzyl)-2-oxo-2,4,5,6,7,7a-hexahydro-thieno[3,2-c]pyridin der Formel (II) zu erhalten,
**e)** die Verbindung der Formel (II) wird acetyliert und das so erhaltene rohe 5-[2-Cyclopropyl-1-(2-fluorphenyl)-2-oxoethyl]-4,5,6,7-tetrahydrothieno[3,2-c]pyridin-2-yl-acetat (Prasugrel) der Formel (I) wird hergestellt, 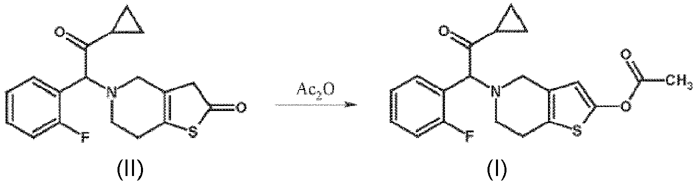
**f)** schließlich wird in einem Reinigungsschritt das rohe Prasugrel in ein gereinigtes Prasugrelprodukt umgewandelt, indem eine Brompentylverunreinigung der Formel (X) 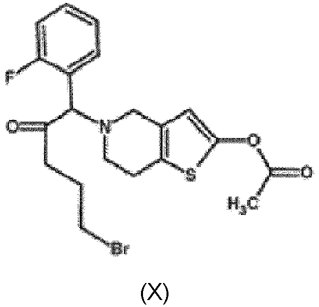 von 5-[2-Cyclopropyl-1-(2-fluorphenyl)-2-oxoethyl]-4,5,6,7-tetrahydrothieno[3,2-c]-pyridin-2-ylacetat (Prasugrel) der Formel (I) 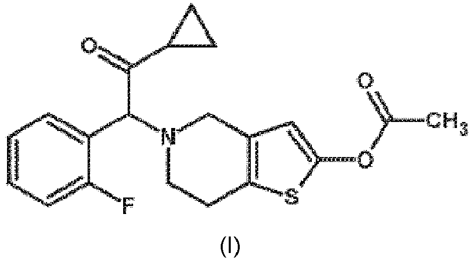 entfernt wird,
wobei die Verunreinigung in ein leicht entfernbares hochpolares quaternäres Ammoniumiodid der Formel (Xb) umgewandelt wird 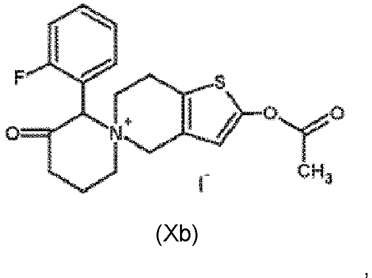 und
wobei die Verwendung von Etherlösungsmitteln in jedem Verfahrensschritt ausgeschlossen ist.

2. Das Verfahren gemäß Anspruch 1, wobei die Brompentylverunreinigung der Formel (X) in einem organischen Lösungsmittel gelöst wird, dann mit einem Überschuss an Natriumiodid umgesetzt wird, wobei das Halogenatom in eine Verbindung (Xa) umgewandelt wird, dann in eine wasserlösliche Verbindung der Formel (Xb), 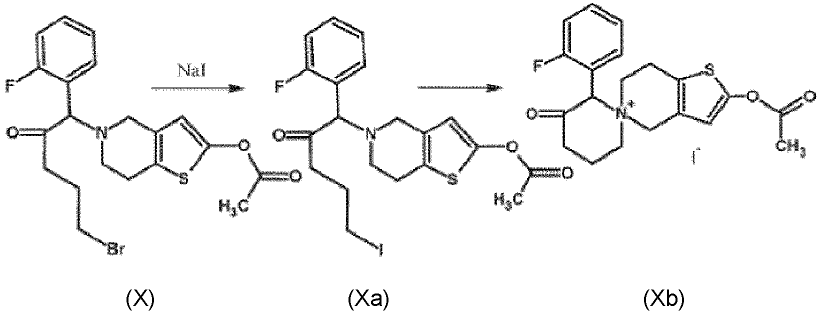 schließlich wird unter Wasserzugabe das gereinigte Prasugrel abfiltriert und getrocknet.

3. Das Verfahren gemäß Anspruch 2, wobei die Reaktion mit Natriumiodid in einem aprotischen Lösungsmittel durchgeführt wird.

4. Das Verfahren gemäß Anspruch 3, wobei das aprotische Lösungsmittel Aceton ist.

5. Das Verfahren gemäß einem der vorhergehenden Ansprüche, wobei die Reaktion in Schritt a) in Gegenwart von Natriumhydridbase durchgeführt wird.

6. Das Verfahren gemäß einem der vorhergehenden Ansprüche, wobei die Reaktion in Schritt a) in einem Gemisch aus Toluol- und Dimethylsulphoxid-Lösungsmitteln durchgeführt wird.

7. Das Verfahren gemäß Anspruch 6, wobei das Gemisch aus Toluol- und Dimethylsulphoxid-Lösungsmitteln in einem Verhältnis von 95:5 vorliegt.

8. Das Verfahren gemäß einem der vorhergehenden Ansprüche, wobei die Bromierung in Schritt b) mit einer äquivalenten Menge Brom bei einer Temperatur in einem Bereich von 24-27 °C durchgeführt wird, der gebildete Bromwasserstoff bei einer Temperatur von 0-5 °C unter Verwendung einer Base in der wässrigen Phase entfernt wird und 2-Brom-1-cyclopropyl-2-(2-fluorphenyl)ethanon durch Eindampfen der organischen Phase erhalten wird.

9. Das Verfahren gemäß einem der vorhergehenden Ansprüche, wobei die Lithiierung in Schritt c) in Toluol unter Verwendung einer tertiären Aminkatalyse durchgeführt wird.

10. Das Verfahren gemäß Anspruch 9, wobei die Lithiierung gemäß Schritt c) in Gegenwart von N,N,N',N'-Tetramethylethylendiamin durchgeführt wird.

11. Das Verfahren gemäß Anspruch 9 oder 10, wobei die Lithiierung gemäß Schritt c) bei einer Temperatur im Bereich von 0-5 °C durchgeführt wird.

## Revendications

1. Procédé pour la préparation d'acétate de 5-[2-cyclopropyl-1-(2-fluorophényl)-2-oxoéthyl]-4,5,6,7-tétrahydrothiéno[3,2-c]pyridin-2-yle (prasugrel) de Formule (I) 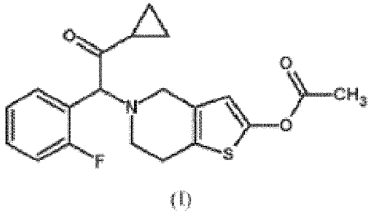 comportant les étapes suivantes :
a) l'acide (2-fluorophényl)-acétique de Formule (VI) est mis à réagir en la présence d'une base avec l'ester d'éthyle de l'acide cyclopropanecarboxylique de Formule (VII), 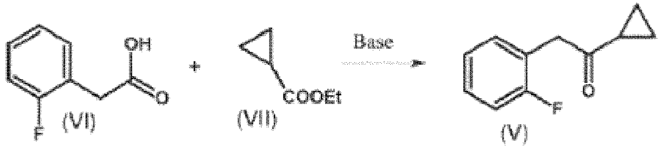
b) puis la 1-cyclopropyl-2-(2-fluorophényl)éthanone de Formule (V) obtenue est bromée pour obtenir la base de 2-bromo-1-cyclopropyl-2-(2-fluorophényl)éthanone de Formule (III), 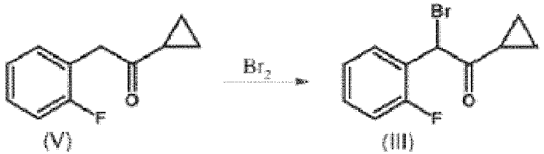
c) la N-trityl-4,5,6,7-tétrahydrothiéno[2,3-c]pyridine de Formule (VIII) est mise à réagir avec de l'hexyllithium, puis avec du borate de tributyle, finalement avec du peroxyde d'hydrogène, 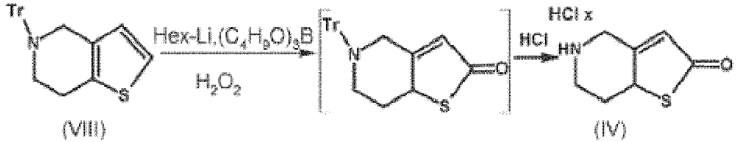 pour former le chlorhydrate de 5,6,7,7a-tétrahydrothiéno[3,2-c]pyridine-2(4H)-one de Formule (IV),
d) la base de 2-bromo-1-cyclopropyl-2-(2-fluorophényl)éthanone de Formule (III), en la présence de carbonate de sodium, est mise à réagir avec le chlorhydrate de 5,6,7,7a-tétrahydrothiéno[3,2-c]pyridine-2(4H)-one de Formule (IV), 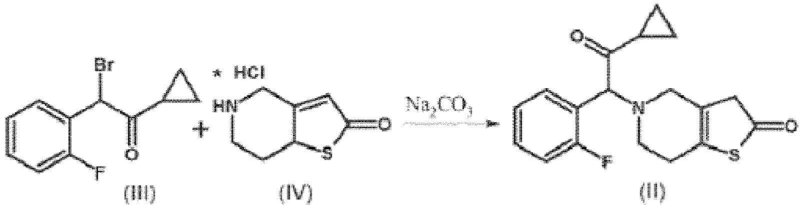 pour obtenir la 5-[α-cyclopropylcarbonyl-2-fluorobenzyl]-2-oxo-2,4,5,6,7,7a-hexahydrothiéno[3,2-c]pyridine de Formule (II),
e) le composé de Formule (II) est acétylé et l'acétate de 5-[2-cyclo-propyl-1-(2-fluorophényl)-2-oxoéthyl]-4,5,6,7-tétrahydrothiéno[3,2-c]pyridin-2-yle (prasugrel) de Formule (I) brut ainsi obtenu est préparé, 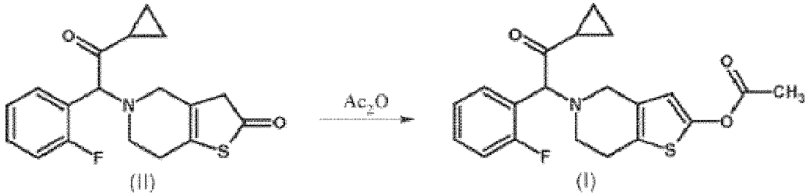
f) finalement, dans une étape de purification, le prasugrel brut est converti en un produit de prasugrel purifié en éliminant une impureté de bromopentyle de Formule (X) 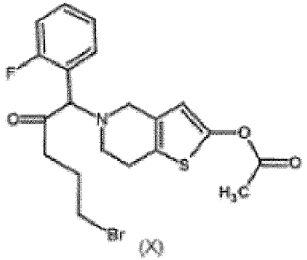 de l'acétate de 5-[2-cyclo-propyl-1-(2-fluorophényl)-2-oxoéthyl]-4,5,6,7-tétrahydrothiéno[3,2-c]pyridin-2-yle (prasugrel) de Formule (I) 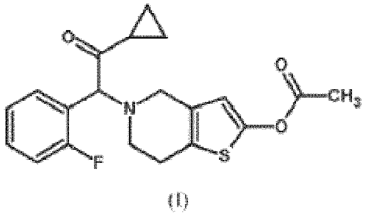 ladite impureté étant convertie en un iodure d'ammonium quaternaire à polarité élevée facilement éliminable de Formule (Xb) 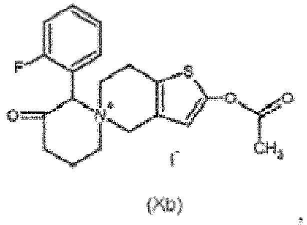 et
l'utilisation de solvants éthérés étant exclue de chaque étape du procédé.

2. Procédé selon la revendication 1, l'impureté de bromopentyle de Formule (X) étant dissoute dans un solvant organique, puis mise à réagir avec un excès d'iodure de sodium, convertie par un changement d'atome d'halogène en un composé (Xa), puis en un composé soluble dans l'eau de Formule (Xb), 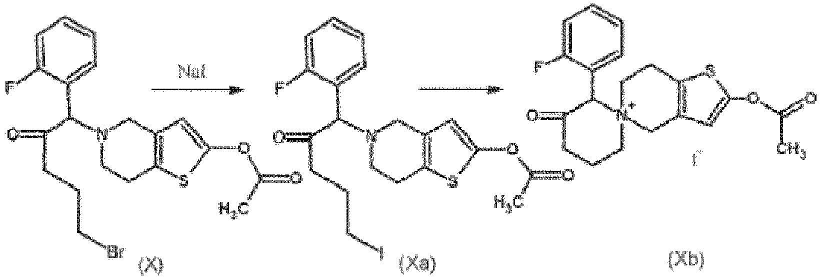 finalement, avec ajout d'eau, le prasugrel purifié est filtré et séché.

3. Procédé selon la revendication 2, la réaction avec de l'iodure de sodium étant réalisée dans un solvant aprotique.

4. Procédé selon la revendication 3, le solvant aprotique étant l'acétone.

5. Procédé selon l'une quelconque des revendications précédentes, la réaction de l'étape a) étant réalisée en la présence d'une base de type hydrure de sodium.

6. Procédé selon l'une quelconque des revendications précédentes, la réaction de l'étape a) étant réalisée dans un mélange de solvants de toluène et de diméthylsulfoxyde.

7. Procédé selon la revendication 6, le mélange de solvants de toluène et de diméthylsulfoxyde se trouvant dans un mélange de rapport 95 : 5.

8. Procédé selon l'une quelconque des revendications précédentes, la bromation de l'étape b) étant réalisée avec une quantité équivalente de brome à une température dans une plage de 24 à 27 °C, le bromure d'hydrogène formé étant éliminé à une température de 0 à 5 °C en utilisant une base dans la phase aqueuse, et la 2-bromo-1-cyclopropyl-2-(2-fluorophényl)éthanone étant obtenue par évaporation de la phase organique.

9. Procédé selon l'une quelconque des revendications précédentes, la lithiation selon l'étape c) étant réalisée dans le toluène en utilisant une catalyse par une amine tertiaire.

10. Procédé selon la revendication 9, la lithiation selon l'étape c) étant réalisée en la présence de N,N,N',N'-tétraméthyléthylène-diamine.

11. Procédé selon la revendication 9 ou 10, la lithiation selon l'étape c) étant réalisée à une température dans la plage de 0 à 5 °C.
